Europäisches Patentamt

European Patent Office

Office européen des brevets

(11) Publication number: **0 210 917**
**B1**

(12) **EUROPEAN PATENT SPECIFICATION**

(45) Date of publication of patent specification: **05.12.90**

(51) Int. Cl.⁵: **C 08 B 11/20,** A 61 K 9/36

(21) Application number: **86401621.7**

(22) Date of filing: **21.07.86**

(54) A method for the preparation of a cellulose ether having a decreased degree of polymerization.

(30) Priority: **24.07.85 JP 163549/85**

(43) Date of publication of application:
**04.02.87 Bulletin 87/06**

(45) Publication of the grant of the patent:
**05.12.90 Bulletin 90/49**

(84) Designated Contracting States:
**BE CH DE FR GB IT LI SE**

(56) References cited:
**DE-A-2 726 780**
**US-A-1 679 943**
**US-A-1 943 461**
**US-A-3 391 135**

(73) Proprietor: **Shin-Etsu Chemical Co., Ltd.**
**6-1, Ohtemachi 2-chome**
**Chiyoda-ku Tokyo 100 (JP)**

(72) Inventor: **Chiba, Tohru**
**2-14-45, Gochi**
**Joetsu-shi Niigata-ken (JP)**
Inventor: **Muto, Hiroaki**
**2-14-45, Gochi**
**Joetsu-shi Niigata-ken (JP)**
Inventor: **Maruyama, Kazumasa**
**2-1-4-15, Minato-machi**
**Joetsu-shi Niigata-ken (JP)**
Inventor: **Hatayama, Atsushi**
**3-6-22, Kasuga-shinden**
**Joetsu-shi Niigata-ken (JP)**
Inventor: **Nishiyama, Yuichi**
**177-42, Oaza Nishifukushima Kubiki-mura**
**Nakakubiki-gun Niigata-ken (JP)**

(74) Representative: **Armengaud Ainé, Alain et al**
**Cabinet ARMENGAUD AINE 3 Avenue Bugeaud**
**F-75116 Paris (FR)**

# EP 0 210 917 B1

**Description**

The present invention relates to a method for the preparation of a cellulose ether having a decreased average degree of polymerization from a starting cellulose ether having a higher degree of polymerization. More particularly, the invention relates to a method for the preparation of a water-soluble cellulose ether of high whiteness having such a decreased average degree of polymerization that a 2% by weight aqueous solution thereof has a viscosity of 20 centipoise (2 · $10^{-2}$ Pa.s.) or lower at 20°C starting from a cellulose ether having a higher degree of polymerization.

As is known, water-soluble cellulose ethers having a relatively low average degree of polymerization or molecular weight are useful, for example, as a film-coating material on solid medicament forms, e.g. pills and tablets, binder of such solid medicament forms, thickening agent of various pasty materials and so on. Several methods are proposed and industrially undertaken for the preparation of such low-molecular water-soluble cellulose ether products. For example, the etherification reaction of an alkali cellulose, which is obtained by the reaction of sodium hydroxide on the starting cellulose pulp, is preceded by a process of air oxidation to give a low-molecular alkali cellulose to be reacted with the etherifying agent. Alternatively, a powdery cellulose ether having a relatively high average degree of polymerization is reacted with gaseous hydrogen chloride or an aliphatic alcohol containing hydrogen chloride absorbed therein (see Japanese Patent Publication 48-41037 US—A—3 391 135) or with hydrogen peroxide (see Japanese Patent Publication 45-678) to cause degradation of the cellulose molecules. US—A—1679943 discloses a method for reducing the molecular weight of cellulose ethers by refluxing for about 15 to 40 hours with (inter alia) dilute HCl. US—A—1 943 461 also discloses an analogous process for molecular weight reduction by treating the cellulose ether with several times its weight of dilute acid, including HCl, at 115—160°C.

These prior art methods have some disadvantages that the yield of the desired product is not always sufficiently high, that the product is sometimes colored in yellow to brown and that the hydrogen peroxide absorbed in the product may react with the medicament ingredients. Colored cellulose ether products can of course be decolorized or bleached to have an increased whiteness by a suitable method such as the method of reacting hydrogensulfite ions in an aqueous alcoholic mixture (see Japanese Patent Publication 46-41628) or reacting sulfur dioxide (see Japanese Patent Kokai 52-152985). These methods are practically not advantageous due to the possibility of the sulfur compounds remaining in the product as an impurity even by setting aside the problem of troublesomeness of the treatment process.

An object of the present invention is therefore to provide a method for the preparation of a cellulose ether of a relatively low average molecular weight having markedly high whiteness without the above described problems and disadvantages in the prior art methods. The object of the invention is, particularly, to provide a method for the preparation of a cellulose ether product which gives a 2% by weight aqueous solution having a viscosity of 20 centipoise (2 · $10^{-2}$ Pa.s) or lower at 20°C.

Thus, the method of the present invention for the preparation of a water-soluble cellulose ether having a decreased average degree of polymerization comprises:

(a) contacting a starting cellulose ether in a powdery form with an aqueous solution of hydrogen chloride in such an amount that the amount of the hydrogen chloride is in the range from 0.1 to 1.0% by weight based on the starting cellulose ether and the amount of watsr is in the range from 3 to 8% by weight based on the overall amount of the starting cellulose ether and the aqueous solution of hydrogen chloride at a temperature in the range from 40 to 85°C; and

(b) removing the hydrogen chloride from the mixture of the powdery cellulose ether and the aqueous solution of hydrogen chloride.

The above described method of the present invention is applicable to a variety of water-soluble cellulose ethers as the starting material including alkyl and hydroxyalkyl celluloses such as methyl cellulose, hydroxyethyl cellulose, hydroxypropyl cellulose and the like and hydroxyalkyl alkyl celluloses such as hydroxyethyl methyl cellulose, hydroxyethyl ethyl cellulose, hydroxypropyl methyl cellulose, hydroxybutyl methyl cellulose and the like. In view of the object of the invention to obtain a low-molecular cellulose ether product, the starting cellulose ether should have a relatively high degcee of polymerization. For example, the starting cellulose ether should give a 2% by weight aqueous solution having a viscosity of at least 20 centipoise (2 · $10^{-2}$ Pa.s) up to several hundreds of centipoise (several $10^{-1}$ Pa.s) at 20°C. These cellulose ethers are usually obtained in a powdery form and can be used as such without further pulverization before the treatment according to the inventive method. The starting cellulose ether should preferably have a particle size distribution as fine as desired of the resultant low-molecular cellulose ether product in order to obtain a full effect of the treatment according to the inventive method.

The starting powdery cellulose ether above mentioned is brought into contact with an aqueous solution of hydrogen chloride. The amount of the aqueous solution of hydrogen chloride should be limited in such a range that from 0.1 to 1.0% by weight of hydrogen chloride is provided based on the amount of the starting cellulose ether. When the amount of hydrogen chloride is too small, the reaction velocity is unduly low so that the desired decrease in the average degree of polymerization of the cellulose ether can be achieved only by taking an unduly long time. When the amount of hydrogen chloride is too large, on the other hand, the reaction velocity would be so large that good control of the reaction can hardly be achieved in addition to the problem that the resultant product can be freed from residual hydrogen chloride only by a

2

long time of removing procedure or may contain an increased amount of chloride ions as an impurity even after a prolonged procedure of removal.

Another important factor in the treatment is the amount of water contained in the mixture of the starting cellulose ether and the aqueous solution of hydrogen chloride. Namely, the water content in the mixture should be in the range from 3 to 8% by weight or, preferably, from 5 to 8% by weight based on the overall amount of the starting cellulose ether and the aqueous solution of hydrogen chloride. When the amount of water is too small corresponding to a high concentration of hydrogen chloride in the solution, the reaction velocity is somewhat increased accordingly but the resultant product is sometimes colored yellow. When the amount of water is too large, on the other hand, agglomerates of the cellulose ether particles may be formed in addition to the decrease in the reaction velocity taking an unduly long time for completion of the reaction. A commercially available product of cellulose ethers usually contains from about 0.5 to about 2.5% by weight of moisture so that this amount of water in the starting cellulose ether should be taken into calculation of the amount and/or concentration of the aqueous solution of hydrogen chloride with which the starting cellulose ether powder is brought into contact. The aqueous solution of hydrogen chloride is added to the starting cellulose ether powder contained in a suitable blending machine either by spraying or in a dropwise manner while the blending machine is running so that the cellulose ether powder is brought into intimate contact with the aqueous acid solution.

The temperature at which the starting cellulose ether is contacted with the aqueous solution of hydrogen chloride should be in the range from 40 to 85°C or, preferably, from 60 to 80°C. The reaction of decreasing the average degree of polymerization is usually complete within 4 hours although the length of the reaction time depends on the desired average degree of polymerization of the product. After completion of the reaction, the hydrogen chloride and water should be removed from the product. This removal of hydrogen chloride and water can be performed by any conventional method such as heating under reduced pressure. It is an alternative way of removing hydrogen chloride that the product after the reaction is admixed with a small amount of a weakly alkaline powdery material, such as sodium hydrogencarbonate, or brought into contact with ammonia gas to neutralize the hydrogen chloride if the small amount of sodium chloride or ammonium chloride formed by the neutralization reaction is permissible in the product.

According to the inventive method, the degree of polymerization can be efficiently decreased in a simple and convenient process without the disadvantages of noticeable yellowing and remaining impurities such as oxidizing agents, organic solvents and sulfur compounds so that the cellulose ether produces obtained by the inventive method are very useful as a coating agent for solid enhancement forms of which high whiteness is particularly desirable.

In the following, the method of the present invention is, described in more detail by way of examples, in which the viscosity of the aqueous solution of cellulose ethers is given with the value obtained by the measurement at 20°C for a 2% by weight aqueous solution in each occurrence.

Example 1 (Experiments No. 1 to No. 5).

In each of Experiments No. 2 to No. 5, into a Henschel mixer of 10 liter capacity was taken 1 kg of a powdery hydroxypropyl methyl cellullose, of which the aqueous solution had a viscosity of 210 centipoise $(2,1 \cdot 10^{-1}$ Pa.s), containing 10% by weight of hydroxypropoxyl groups, 29% by weight of methoxyl groups and 0.9% by weight of moisture and the mixer was driven at a velocity of 200 rpm. An aqueous solution of hydrogen chloride in a varied concentration as indicated in Table 1 below was sprayed to the cellulose ether powder in the running mixer in such an amount that 3 g of hydrogen chloride were introduced. After the cellulose ether powder was uniformly wet with the acid solution, a 500 g portion of the powder was taken in a glass-made vessel of 2 liter capacity and the vessel was tumbled in a water bath kept at 75°C until the viscosity of the cellulose ether had dropped to about 5 to 7 centipoise (5 to $7 \cdot 10^{-3}$ Pa.s). After a length of time indicated in Table 1, the vessel was evacuated at 75°C for 30 minutes so as to remove the hydrogen chloride and water under a reduced pressure of 80 mmHg from the cellulose ether product. The cellulose ether powder thus dried and freed from hydrogen chloride was admixed and thoroughly blended with 2 g of sodium hydrogencarbonate to neutralize any trace amount of hydrogen chloride.

In Experiment No. 1 undertaken for comparative purpose, dry hydrogen chloride gas was blown into the glass vessel kept in the water bath at 75°C in place of addition of the aqueous solution of hydrogen chloride until the amount of hydrogen chloride blown thereinto had reached the same amount as that in the other experiments using the aqueous acid solution.

The thus obtained cellulose ether products were each subjected to the measurement of the viscosity of an aqueous solution in a usual manner and evaluation of the yellowness YI for a 2% by weight aqueous solution in a glass cell of 50 mm optical path using a color computer (Model SM-2, manufactured by Suga Test Instruments Co.) to give the results shown in Table 1, in which Experiments No. 1, No. 2 and No. 5 were for comparative purpose and Experiments No. 3 and No. 4 were for the invention. In Experiment No. 5, some agglomerates were found in the cellulose ether powder taken out of the tumbling vessel.

EP 0 210 917 B1

TABLE 1

| Experiment No. | HCl concentration of acid solution, % | Overall moisture content, % | Reaction time, hours | Viscosity of aqueous solution, Pa.s | Yellowness, YI |
|---|---|---|---|---|---|
| 1 | (dry gas) | 0.9 | 1.6 | $6.1 \cdot 10^{-3}$ | 21 |
| 2 | 20 | 2.1 | 2.0 | $5.7 \cdot 10^{-3}$ | 17 |
| 3 | 10 | 3.5 | 2.0 | $6.4 \cdot 10^{-3}$ | 13 |
| 4 | 5 | 6.2 | 3.0 | $6.7 \cdot 10^{-3}$ | 13 |
| 5 | 3 | 9.6 | 5.0 | $7.7 \cdot 10^{-3}$ | 13 |

Example 2

The experimental procedure was substantially the same as in Example 1, in which 40 g of a 10% aqueous solution of hydrogen chloride were sprayed to 1 kg of a powdery methyl cellulose, of which the aqueous solution had a viscosity of 380 centipoise ($3,8 \cdot 10^{-1}$ Pa.s), containing 29.7% by weight of methoxyl groups and 1.2% by weight of moisture and the powder was introduced into a vessel which was tumbled for 2 hours in a water bath kept at 70°C followed by a post-treatment in the same manner as in Example 1. The thus obtained methyl cellulose powder gave an aqueous solution having a viscosity of 15 centipoise ($1,5 \cdot 10^{-2}$ Pa.s) and the yellowness YI thereof was 11.

For comparison, 500 g of the same methyl cellulose powder were taken in a vessel which was tumbled at 70°C for 100 minutes while dry hydrogen chloride gas was blown thereinto in an amount of 2 g over the above mentioned period. The thus obtained methyl cellulose powder gave an aqueous solution having a viscosity of 14 centipoise ($1,4 \cdot 10^{-2}$ Pa.s) and the powder was colored in a clearly deeper yellow than above with a yellowness YI of 15.

Example 3

In a similar manner to the preceding examples, 36 g of a 11% aqueous solution of hydrogen chloride was sprayed to 1 kg of a powdery hydroxyethyl cellulose, of which the aqueous solution had a viscosity of 450 centipoise ($4,5 \cdot 10^{-1}$ Pa.s), containing 25% by weight of ethoxyl groups and 0.8% by weight of moisture. The powder was introduced in a vessel which was tumbled for 2 hours in a water bath kept at 75°C followed by the post-treatment in the same manner as in the preceding examples. The resultant ethyl cellulose powder gave an aqueous solution having a viscosity of 3.7 centipoise ($3,7 \cdot 10^{-3}$ Pa.s) and the yellowness YI thereof was 16.

For comparison, 500 g of the same ethyl cellulose powder were taken in a vessel which was tumbled in the same manner as above while 3.5 g of dry hydrogen chloride gas were blown thereinto over the length of time. The thus obtained powdery ethyl cellulose gave an aqueous solution having a viscosity of 3.5 centipoise ($3,5 \cdot 10^{-3}$ Pa.s) but it was colored in clearly deeper yellow than above with a yellowness YI of 27.

**Claims**

1. A method for the preparation of a water-soluble cellulose ether having a decreased average degree of polymerization which comprises:

(a) contacting a starting cellulose ether in a powdery form with an aqueous solution of hydrogen chloride in such an amount that the amount of the hydrogen chloride is in the range from 0.1 to 1.0% by weight based on the starting cellulose ether and the amount of water is in the range from 3 to 8% by weight based on the overall amount of the starting cellulose ether and the aqueous solution of hydrogen chloride at a temperature in the range from 40 to 85°C; and

(b) removing the hydrogen chloride from the mixture of the powdery cellulose ether and the aqueous solution of hydrogen chloride.

2. The method as claimed in Claim 1 wherein the amount of water is in the range from 5 to 8% by weight based on the overall amount of the starting cellulose ether and the aqueous solution of hydrogen chloride.

3. The method as claimed in Claim 1 wherein the temperature at which the starting cellulose ether is contacted with the aqueous solution of hydrogen chloride is in the range from 60 to 80°C.

**Patentansprüche**

1. Verfahren zur Herstellung eines wasserlöslichen Celluloseethers mit einem verminderten durchschnittlichen Polymerisationsgrad, wobei man:

(a) einen Celluloseether, den man pulverförmig einsetzt, bei einer Temperatur von 40 bis 85°C mit einer solchen Menge einer wässrigen Chlorwasserstofflösung in Kontakt bringt, daß die Menge an Chlorwasserstoff 0,1 bis 1,0 Gew.-%, bezogen auf den eingesetzten Celluloseether, und die Wassermenge 3 bis 8 Gew.-%, bezogen auf die Gesamtmenge aus dem eingesetzte Celluloseether und der wässrigen Chlorwasserstofflösung, beträgt und

(b) den Chlorwasserstoff aus der Mischung aus dem pulverförmigen Celluloseether und der wässrigen Chlorwasserstofflösung entfernt.

2. Verfahren nach Anspruch 1, in dem die Wassermenge 5 bis 8 Gew.-%, bezogen auf die Gesamtmenge aus dem eingesetzten Celluloseether und der wässrigen Chlorwasserstofflösung, beträgt.

3. Verfahren nach Anspruch 1, in dem die Temperatur, bei der der eingesetzte Celluloseether mit der wässrigen Chlorwasserstofflösung in Kontakt gebracht wird, 60 bis 80°C beträgt.

**Revendications**

1. Procédé de préparation d'un éther cellulosique hydrosoluble, ayant un degré de polymérisation moyen réduit, qui comprend les étapes consistant à:

(a) mettre en contact un éther cellulosique de départ sous forme de poudre avec une solution aqueuse de chlorure d'hydrogène en une proportion telle, que la proportion du chlorure d'hydrogène est dans la gamme de 0,1 à 1,0% en poids par rapport à l'éther cellulosique de départ, et la proportion de l'eau est dans la gamme de 3 à 8% en poids par rapport à la quantité totale de l'éther cellulosique de départ et de la solution aqueuse de chlorure d'hydrogène, à une température dans la plage de 40 à 85°C; et

(b) éliminer le chlorure d'hydrogène du mélange de l'éther cellulosique en poudre et de la solution aqueuse de chlorure d'hydrogène.

2. Procédé selon la revendication 1, dans lequel la proportion d'eau est dans la gamme de 5 à 8% en poids par rapport à la quantité totale de l'éther cellulosique de départ, et de la solution aqueuse de chlorure d'hydrogène.

3. Procédé selon la revendication 1, dans lequel la température à laquelle l'éther cellulosique de départ est mis en contact avec la solution aqueuse de chlorure d'hydrogène est dans la gamme de 60 à 80°C.